# EUROPÄISCHE PATENTSCHRIFT

(11) **EP 1 388 958 B1**
(45) Veröffentlichungstag und Bekanntmachung des Hinweises auf die Patenterteilung: **16.03.2011**
(21) Anmeldenummer: 03017901.4
(22) Anmeldetag: 05.08.2003
(51) Int. Cl.: H04B 10/155, H04B 10/22, A61B 18/22, A61B 18/24, A61C 1/00, B23K 26/04

(54) **Laserübertragungssystem**
Laser communication system
Système de communication par laser

(30) Priorität: 07.08.2002 DE 10236175
(43) Veröffentlichungstag der Anmeldung: 11.02.2004
(73) Patentinhaber: Dornier MedTech Laser GmbH, 82234 Wessling (DE)
(72) Erfinder: Illich, Wolfgang, 82234 Wessling (DE); Austen, Jürgen, 85356 Freising (DE); Hiereth, Werner, 82205 Gilching/Geisenbrunn (DE)
(74) Vertreter: Grünecker, Kinkeldey, Stockmair & Schwanhäusser Anwaltssozietät

(56) Entgegenhaltungen:
- WO-A-02/101957
- US-A- 4 956 877
- US-A- 5 350 377
- US-A- 5 968 035

## Beschreibung

Die vorliegende Erfindung bezieht sich im Allgemeinen auf ein Lasersystem mit fasergebundener Kommunikation und im Speziellen auf ein Lasersystem für Anwendungen im medizinischen Bereich.

Laserlicht hat sich nicht nur in der Forschung, sondern auch in vielen Bereichen des täglichen Lebens als steuerbare Lichtquelle durchgesetzt. Ausschlaggebend hierfür ist, dass mit einem Laser eine monochromatische Lichtquelle mit kohärenter Strahlung zur Verfügung steht, deren Strahlung sich aufgrund der geringen Divergenz sehr gut bündeln lässt. Der Laser hielt dabei nicht nur Einzug in der Telekommunikation und der Unterhaltungselektronik, sondern auch in der Materialbearbeitung und in der Medizin. Für letzteres Gebiet war ausschlaggebend, dass mit einem Laser ein Instrument zur Verfügung steht, bei dem zum einen energiereiche Strahlung zur gezielten Erhitzung von Gewebe und zur Zerstörung von körpereigenen Steinen und zum anderen monochromatischen Strahlung zur selektiven Anregung fotochemischer Prozesse eingesetzt werden kann. Dabei ist zwischen dem Einsatz des Lasers als Hilfsinstrument im Rahmen einer größeren Operation oder dem Einsatz des Lasers als eigentliches Therapieverfahren zu unterscheiden.

Im chirurgischen Bereich kommt der Laser vor allem wegen der blutstillenden Wirkung, der Möglichkeit der präzisen Handhabung und der Verringerung der Instrumentenzahl im Operationsfeld zum Einsatz. Berührungsfreie Gewebeabtragung und minimale Traumatisierung des umliegenden Gewebes durch kräftefreies Bearbeiten des Gewebes sind dabei wesentliche Vorteile der Laseranwendung.

Ein weiteres Einsatzgebiet der Lasertechnologie in der Medizin ist die Behandlung von Körperoberflächen. Hierbei wird der Laser zum einen für das Abtragen oder Koagulieren von Haut- und Hautanhangsgebilden verwendet, zum anderen für die Therapie von intrakutanen Gefäßveränderungen und Missbildungen. So werden Hauttumore heute vorzugsweise mit dem Nd:YAG-Laser koaguliert oder alternativ mit einem CO₂-Laser abgetragen. Gutartige Pigmentanomalien werden mit Alexandrit- oder Argonlaser behandelt. Zum Entfernen von Tätowierungen sowie zur Haarentfernung werden ebenfalls Alexandritlaser und gepulste Nd:YAG-Laser verwendet.

Auch in der Endoskopie ist der Laser unentbehrlich geworden. Laserlicht, das über Lichtleiter an die Stelle der Applikation geleitet wird, kann hier zu einer weiteren Verkleinerung und damit zu einer weiteren Flexibilisierung des endoskopischen Operierens führen. Durch die Weiterentwicklung und vor allem durch die Miniaturisierung der lnstrumente sowie durch eine Verfeinerung der flexiblen Endoskope erschließt sich eine Reihe von neuen Einsatzgebieten für die Lasertechnologie, in denen bislang konventionell operiert werden musste oder die keiner minimal invasiven Therapie zugänglich waren.

Ein Signalmodulationssystem, in dem eine erste Station unmoduliertes Licht durch einen Lichtleiter an eine zweite Station schickt, welche durch partielle Reflektion moduliertes Licht durch denselben Lichtleiter an die erste Station zurückschickt, wird in US-Patent 4956877 beschrieben.

Eine Zahnbehandlungsmethode, bei der am Zahn reflektiertes Laserlicht verwendet wird, um die Laserquelle für eine verbesserte Zahnbehandlung nachzuregeln wird im US-Patent 5968035 diskutiert.

Die immer zahlreicheren Anwendungsgebiete der Lasertechnologie in der Medizin führten zur Entwicklung von technisch immer mehr ausgereiften Laserkonstruktionen und entsprechenden Systemkonzepten, die die Handhabung der Lasersysteme erleichterten und verbesserten, bzw. wiederum neue Einsatzgebiete erschlossen.

Diese Entwicklung der medizinischen Lasersysteme setzt sich nun fort bis hin zu "intelli-genten" Systemen. In DE4025851 wird ein solches "intelligentes" Lasersystem beschrieben, bei dem remittierte Strahlung, die bei der Bearbeitung von Material mittels Laserlicht entsteht, über ein Transmissionssystem für das Laserlicht zu einem Detektor übertragen wird. Der Detektor ermittelt die Intensität der remittierten Strahlung. Über ein derartiges Lasersystem kann erreicht werden, dass ungewollte Gewebeschädigungen minimiert werden, da die Laserleistung über die Detektion der Intensität der remittierten Strahlung automatisch geregelt wird.

In diesem Zusammenhang kommt dem Einsatz von flexiblen, optischen Transmissionssystemen für die erzeugte Laserstrahlung eine wesentliche Bedeutung zu, denn für die Applikation der Laserstrahlung auf das zu behandelnde Gewebe muss die Entfernung zwischen dem Lasergeräteausgang und dem Patienten überbrückt werden. Die medizinischen Lasersysteme bestehen daher typischerweise aus einem stationären oder mobilen Lasergerät, einer Strahlführung, optischen Endgeräten und Zubehör für spezielle medizinische Applikationen. Für die Übertragung von sichtbarem Laserlicht und den angrenzenden Spektralbereichen von ca. 0,3 bis 2,1 µm werden flexible Glas- bzw. Quarzfasern verwendet. In den Spektralbereichen 0,19 - 0,3 µm (Eximer-Laser) und 3 - 10 µm (Erbium- und CO₂-Laser) finden spezielle Lichtleiter oder Spiegelgelenkarme Anwendung. Dabei werden besonders hohe Anforderungen an die Lichtleiter bei der Übertragung gepulster, energiereicher Laserstrahlung zur Laserlithotripsie gestellt. Die gute Handhabbarkeit und Flexibilität dieser Transmissionssysteme ist von entscheidender Bedeutung für den Einsatz der Lasersysteme.

Bei allen Anwendungsmöglichkeiten des Lasers in der Medizin ist die Handhabbarkeit der Laserstrahlung von wesentlicher Bedeutung. Flexibilität, Ergonomie und Funktionalität spielen dabei genauso eine wesentliche Rolle, wie Sicherheit, Zuverlässigkeit und Präzision. Sie sind entscheidend dafür, ob bei einer Behandlung die Vorteile des Lasers auch zum Tragen kommen.

Von wesentlicher Bedeutung hierfür ist, wie das Handstück eines Lasersystems, das mit einem Spiegelgelenkarm oder einer flexiblen Glasfaser an den Lasergeräteausgang gekoppelt ist, handhabbar und bedienbar ist. Dies beinhaltet Funktionalitäten wie z. B. eine stufenlose Verstellung der Spotgröße des Laserfokus. Bisher musste man nach Verstellung der Spotgröße die Laserstärke manuell am Lasergerät nachregeln. Bei neueren Generationen von Lasersystemen kann nun die Stärke des Lasers über eine Fernbedienung angepasst werden. Für die Datenübertragung von einem Laserhandstück zum Lasergerät werden dazu entweder zusätzliche elektrische Kabel vom Handstück zum Laser oder eine RF-Funkübertragung verwendet. Eine weitere Möglichkeit ist eine Infrarot-Übertragung, die entweder gerichtet oder diffus ist. Alle drei Übertragungsmöglichkeiten haben gewisse spezifische Nachteile bezüglich der Handlichkeit, des Stromverbrauchs und der Wirksamkeit des Verfahrens oder führen zu Problemen bei einer weltweiten Zulassung des Lasersystems.

Es ist daher die Aufgabe der vorliegenden Erfindung, ein Lasersystem anzugeben, das einen sicheren Datenaustausch zwischen Handstück und Stationärgerät ermöglicht, die ergonomischen Anforderungen an das Handstück berücksichtigt, mit geringen Aufwand in bestehende Lasersysteme zu integrieren ist und keine weiteren Erschwernisse für eine weltweite Zulassung des Lasergeräts verursacht.

Diese Aufgabe wird in erfindungsgemäßer Weise durch den Gegenstand des Anspruchs 1 gelöst. Vorteilhafte Ausgestaltungen der Erfindung sind Gegenstand der Unteransprüche.

Die vorliegende Erfindung baut auf der Erkenntnis auf, dass eine Datenübertragung vom Handstück zum Stationärteil des Lasers mittels Nutzung des Lichtleiters geschehen kann, der für den Therapielaser verwendet wird.

Daher kann durch die erfindungsgemäße Lösung eine Datenkommunikation zwischen Handstück und Stationärteil des Lasersystems realisiert werden, bei der weder zusätzliche Kabel zwischen Handstück und Laser notwendig sind noch aufgrund von RF-Komponenten Zulassungsverfahren für eine weltweite Zulassung des Systems negativ beeinflusst werden. Darüber hinaus werden Probleme mit der Zuverlässigkeit der Datenkommunikation wie z. B. bei gerichteter oder diffuser Infrarot-Übertragung vermieden. Des Weiteren bietet die erfindungsgemäße Lösung den Vorteil, dass sie in bestehende Systeme integriert werden kann, vor allem, da sie kaum in die komplizierte Optik des Lasersystems eingreift. Bei bestimmten Lasersystemen können bereits vorhandene Systemkomponenten mitverwendet werden, wodurch der Aufwand für die Integration der Datenübertragung vom Handstück zum Stationärteil des Lasersystems weiter reduziert wird.

Gemäß einer bevorzugten Ausführungsform beinhaltet das Lasersystem eine Einrichtung zur Erzeugung von Laserstrahlung; eine Einkoppeleinrichtung zur Einkopplung der erzeugten Laserstrahlung in einen Lichtleiter und eine Auskoppeleinrichtung zur Auskopplung der Laserstrahlung aus dem Lichtleiter, wobei eine Datensendeeinrichtung an die Ein- oder Auskoppeleinrichtung gekoppelt ist und optische Signale erzeugt, die über die Ein- oder Auskoppeleinrichtung in den Lichtleiter eingekoppelt werden und am anderen Ende des Lichtleiters von einer Datenempfangseinrichtung empfangen werden.

Gemäß einer weiteren bevorzugten Ausführungsform beinhaltet das Lasersystem sowohl in der Einkoppeleinrichtung eine Datenempfangs- und Datensendeeinrichtung als auch bei der Auskoppeleinrichtung, um eine bidirektionale, optische Signalübertragung über den Lichtleiter zu ermöglichen. Dabei dienen die optischen Signale zur Übertragung von Informationen zum Handstück, die insbesondere zur Steuerung der Vorrichtung zur Erzeugung von Laserstrahlung, zur Steuerung der Ein- oder Auskoppeleinrichtung und insbesondere zur Regulierung der Laserleistung verwendet werden.

Gemäß einer besonders bevorzugten Ausführungsform handelt es sich bei dem Lasersystem um ein medizinisches Lasersystem, wobei die an die Auskoppeleinrichtung gekoppelte Datensendeeinrichtung eine LED oder andere Lichtquelle ist, die optische Signale im blauen Spektralbereich erzeugt. Die optischen Signale der LED werden bei der Einkoppeleinrichtung über einen Spiegel mit dielektrischer Beschichtung aus dem Strahlengang des Lasers ausgekoppelt und an eine Datensendeeinrichtung übertragen, die gleichzeitig auch zur Detektion weiterer optischer Signale verwendet werden kann, z. B. zur Messung von reemittierter Strahlung, die durch die Bearbeitung von Material mittels der Laserstrahlung entsteht.

Bei einer Alternative der besonders bevorzugten Ausführungsform der vorliegenden Erfindung weist das Handstück Bedieneinrichtungen auf, die bei Betätigung elektrische Signale erzeugen und an die LED im Handstück weiterleiten, mittels derer die Einrichtung zur Erzeugung von Laserstrahlung gesteuert wird. Über diese Bedieneinrichtung kann vorzugsweise die Laserleistung gesteuert werden.

In einer weiteren Alternative der besonders bevorzugten Ausführungsform der vorliegenden Erfindung ist das Handstück modular aufgebaut und je nach Verwendungszweck des Lasers werden Elemente des Handstücks ergänzt, ausgetauscht oder abgenommen. Die einzelnen Elemente des Handstücks verfügen über eine elektronisch oder optisch lesbare ldentifizierungseinrichtung, die bei der Befestigung der Elemente am Handstück über eine Leseeinheit im Handstück ausgelesen wird und die Daten aus der ldentifizierungseinrichtung an die Datensendeeinrichtung im Handstück weiterleitet und zur Steuerung des Handstücks oder der Einrichtung zur Erzeugung von Laserstrahlung verwendet wird.

Anhand der in der beiliegenden Zeichnungen dargestellten bevorzugten Ausführungsformen wird die Erfindung im Folgenden näher erläutert. Ähnliche oder korrespondierende Einzelheiten sind in den Figuren mit denselben Bezeichnungen versehen. Es zeigen:
- Fig. 1: eine schematische Darstellung eines Lasersystems gemäß der vorliegenden Erfindung,
- Fig. 2: eine schematische Darstellung der Auskoppeleinrichtung sowie des Lichtleiters gemäß einer bevorzugten Ausführungsform der vorliegenden Erfindung,
- Fig. 3: eine schematische Darstellung der Einkoppeleinrichtung sowie des Lichtleiters gemäß einer bevorzugten Ausführungsform der vorliegenden Erfindung,
- Fig. 4: eine schematische Darstellung der Auskoppeleinrichtung sowie des Lichtleiters gemäß einer besonders bevorzugten Ausführungsform der vorliegenden Erfindung,
- Fig. 5: eine schematische Darstellung der Einkoppeleinrichtung sowie des Lichtleiters gemäß einer besonders bevorzugten Ausführungsform der vorliegenden Erfindung,
- Fig. 6: eine schematische Darstellung eines Regelkreises zur Regulierung der Laserleistung gemäß einer besonders bevorzugten Ausführungsform der vorliegenden Erfindung,
- Fig. 7: ein Flussdiagramm für den schematischen Ablauf der Datenkommunikation zwischen dem Stationärteil des Lasersystems und dem Handstück gemäß der besonders bevorzugten Ausführungsform der vorliegenden Erfindung, und
- Fig. 8: ein weiteres Flussdiagramm für den schematischen Ablauf der Datenkommunikation zwischen dem Stationärteil des Lasersystems und dem Handstück gemäß der besonders bevorzugten Ausführungsform der vorliegenden Erfindung.

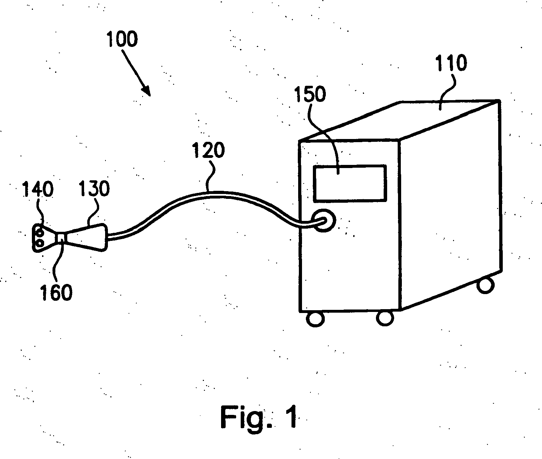

Fig. 1 zeigt anhand einer schematischen Darstellung eine bevorzugte Ausführungsform der vorliegenden Erfindung. Das Lasersystem 100 besteht aus einem stationären oder transportierbaren Lasergerät 110, das eine Einrichtung zur Erzeugung von Laserstrahlung beinhaltet, an das ein flexibler Lichtleiter 120 gekoppelt ist, der mit einem Handstück 130 verbunden ist.

Das Lasergerät ist zur Erzeugung von intensiver Laserstrahlung mit Hochleistungslaserdioden, einer Mikrooptik zum Fokussieren des erzeugten Laserlichts, sowie einer Energieversorgung ausgestattet. Alternativ ist das Lasergerät mit einem Lasermedium, einem Resonator und einer Pumpquelle sowie der entsprechenden Energieversorgung ausgestattet. In diesem Fall finden bevorzugterweise diodengepumpte Festkörperlasermedien Anwendung für die Erzeugung der intensiven Laserstrahlung.

Darüber hinaus verfügt das Lasergerät vorzugsweise über eine Kühleinrichtung sowie einen Systemkontroller, der unter anderem auch die Leistung der Laserstrahlung, die Pulsdauer sowie die Frequenz der Laserpulse regelt. Darüber hinaus sind in das Lasergerät Anzeige- und Bedieneinrichtungen 150 integriert, über die bestimmte Anwendungsmodi sowie Systemeinstellungen ausgewählt werden können. Des Weiteren verfügt das Lasergerät über entsprechende Sicherheitseinrichtungen sowohl für den elektrischen wie auch den optischen Bereich. Vorzugsweise besitzt der Systemkontroller entsprechende Einrichtungen, um die Steuerung und Regelung des Lasersystems über Software-Programme durchführen zu können. Besonders vorteilhaft ist dabei eine Ausführungsform, bei der Software-Programme bei einer Aktualisierung ausgetauscht werden. Bei einer weiteren Alternative der bevorzugten Ausführungsform der vorliegenden Erfindung ist in das Lasergerät eine Ausgabeeinheit für ein Protokoll der Systemeinstellungen integriert bzw. weist das Lasergerät eine Schnittstelle für eine Ausgabeeinheit auf.

Des Weiteren ist in das Lasergerät eine Einkoppeleinrichtung integriert, die die erzeugte Laserstrahlung in den Lichtleiter 120 einkoppelt. Der Lichtleiter ist hierzu fest oder lösbar, vorzugsweise mit einem Präzisionsbajonettverschluss, vorzugsweise in einer festen Position zur Einkoppeleinrichtung befestigt. Alternativ kann zu einem Präzisionsbajonettverschluss ein sogenannter SMA-Konnektor verwendet werden. Der Lichtleiter kann aus einer oder mehreren Plastik-, Glas- oder Quarzglasfasern bestehen. Je nach Wellenlänge der erzeugten Laserstrahlung werden auch dotierte Quarzglasfasern verwendet. Der Lichtleiter ist dafür ausgelegt, hohe Lichtleistungen möglichst verlustfrei transportieren zu können. Er verfügt aus Sicherheitsgründen vorzugsweise über eine entsprechende Ummantelung, die die Fasern vor entsprechender mechanischer Belastung und im Falle eines Bruchs der Fasern vor einem Austreten der Laserstrahlung schützen.

Ähnlich wie das Lasergerät verfügt das Handstück über eine entsprechende Vorrichtung, mittels derer der Lichtleiter fest oder abnehmbar an dem Handstück befestigt werden kann. Entsprechend ist eine Auskoppeleinrichtung in das Handstück integriert, die die erzeugte Laserstrahlung aus dem Lichtleiter auskoppelt und je nach Anwendungsgebiet des Lasersystems an eine entsprechende Ausgabeeinrichtung für den Laserstrahl im Handstück weiterleitet. Hierfür besitzt das Handstück eine entsprechende Austrittsöffnung für die Laserstrahlung. Des Weiteren verfügt das Handstück der bevorzugten Ausführungsform der vorliegenden Erfindung vorzugsweise über entsprechende Bedieneinrichtungen 140, mittels derer bestimmte Funktionen des Lasergerätes gesteuert werden können, beispielsweise die Laserleistung. Zusätzlich sind in dem Handstück eine oder mehrere Anzeigeneinrichtungen angebracht, über die Informationen angezeigt und Einstellungen des Handteils oder des Lasergerätes kontrolliert werden können.

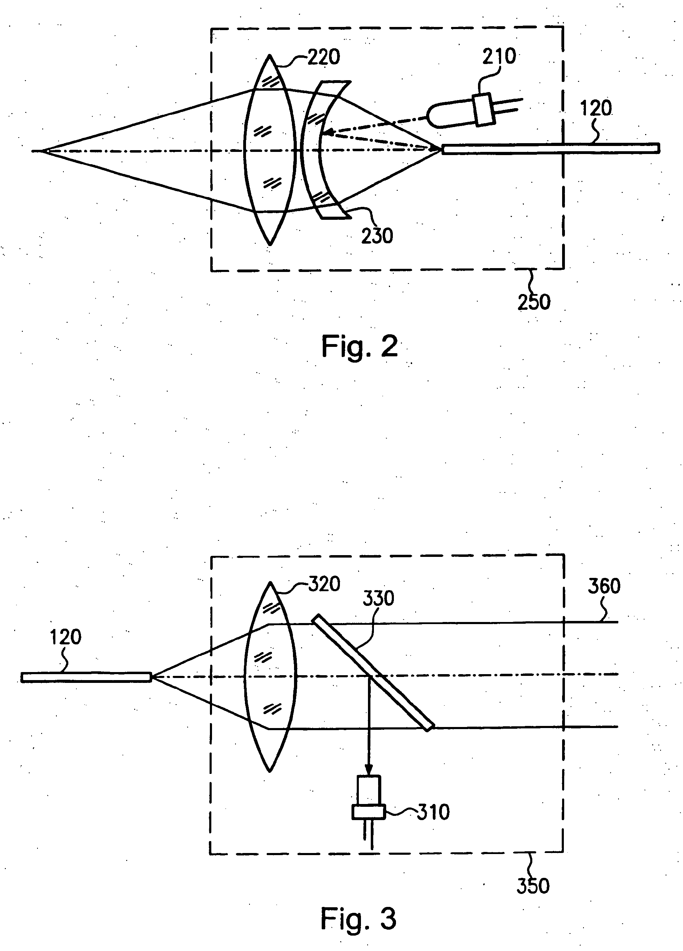

Fig. 2 zeigt anhand einer schematischen Darstellung die Auskoppeleinrichtung 250 einer bevorzugten Ausführungsform der vorliegenden Erfindung. Die aus dem Lichtleiter 120 austretende Laserstrahlung wird mittels eines Linsensystems 220 und 230 gebündelt und an eine Ausgabeeinrichtung im Handstück 130 weitergeleitet. An die Auskoppeleinrichtung ist eine Datensendeeinrichtung 210 gekoppelt, die optische Signale erzeugt und über eine Reflexion an dem Linsensystem in den Lichtleiter einkoppelt. Vorzugsweise besteht die Datensendeeinrichtung aus einer LED, die optische Signale in einem anderen Spektralbereich als die Laserstrahlung emittiert. Alternativ besteht die Datensendeeinrichtung aus anderen Bauelementen zur Erzeugung optischer Signale.

Besonders bevorzugterweise wird bei Lasergeräten, die Laserstrahlung hoher Leistung im IR-Bereich erzeugen, eine LED verwendet, die im blauen Spektralbereich emittiert, um einen genügenden Störabstand der erzeugten optischen Signale zu der Laserstrahlung zu gewährleisten.

Alternativ können für die Datensendeeinrichtungen sowohl LEDs verwendet werden, die in anderen Wellenlängenbereichen emittieren sowie andere Komponenten zur Erzeugung von optischen Signalen. Das Linsensystem ist mit einer dielektrischen Beschichtung versehen, die dahingehend optimiert ist, dass die Reflexion der Laserstrahlung minimiert und damit die Transmission der Laserstrahlung durch das Linsensystem maximiert wird. Alternativ wird die Beschichtung des Linsensystems auch dahingehend optimiert, dass zusätzlich für den Spektralbereich der optischen Signale der Datensendeeinrichtung 210 eine möglichst hohe Reflexion an dem Linsensystem entsteht. Dadurch können die optischen Signale der Datensendeeinrichtung besonders effizient in den Lichtleiter eingekoppelt werden.

Bei der bevorzugten Ausführungsform der vorliegenden Erfindung sind im Handstück 130 mechanische Verstelleinrichtungen angebracht, die an ein Potentiometer oder an elektrische oder optische Inkrementalgeber gekoppelt sind. Das Potentiometer oder die elektrischen oder optischen Inkrementalgeber geben über eine elektronische Einrichtung die aktuellen Einstellungen der mechanischen Verstelleinrichtungen an die Datensendeeinrichtung 210 weiter. Auf diese Weise kann z. B. der Abstand des Handstücks zu einer Oberfläche und damit die Laserspotgröße ermittelt und dementsprechend die Laserleistung nachgeregelt werden. Alternativ kann eine Abstandsmessung statt über eine mechanische Verstelleinrichtung auch über einen berührungslosen Sensor im Handstück erfolgen.

Zur Stromversorgung der Elektronik für die Datensendeeinrichtung 210 sowie für die Bedieneinrichtung 140 notwendige Elektronik verfügt das Handstück über eine Batterie bzw. über eine wiederaufladbare Batterie. Alternativ wird eine kapazitätsgepufferte Energieversorgung verwendet.

Das Handstück muss zum einen ergonomische Anforderungen erfüllen, zum ändern muss es entsprechend ermüdungsfrei handhabbar sein, so dass das Gewicht des Handstücks möglichst gering gehalten werden muss. Daher findet als weitere Alternative für die Stromversorgung eine oder mehrere Photozellen Anwendung, die in das Handstück integriert sind und entweder vorhandenes Raumlicht zur Erzeugung von elektrischer Energie benutzen und/oder einen Teil der reflektierten Laserstrahlung.

Die Position der Datensendeeinrichtung ist so gewählt, dass sie außerhalb der numerischen Apertur (NA) des Lichtleiters liegt. Besonders vorteilhaft ist eine Positionierung der Datensendeeinrichtung bzw. der LED, so dass ein möglichst großer Anteil der am Linsensystem reflektierten optischen Signale der LED bzw. Datensendeeinrichtung innerhalb der numerischen Apertur des Lichtleiters liegen.

Fig. 3 zeigt die schematische Darstellung der Einkoppeleinrichtung 350 einer besonders bevorzugten Ausführungsform der vorliegenden Erfindung. Die im Lasergerät erzeugte Laserstrahlung 360 wird über die Einkoppeleinrichtung 350 mittels eines Linsensystems 320 entsprechend der numerischen Apertur des Lichtleiters in den Lichtleiter 120 eingekoppelt. Eine Datenempfangseinrichtung ist über einen teildurchlässigen dielektrischen Spiegel 330 an die Einkoppeleinrichtung gekoppelt. Der dielektrische Spiegel ist im Strahlengang des Lasers geneigt angebracht, damit von der Datensendeeinrichtung 210 über den Lichtleiter geleitete optische Signale aus dem Strahlengang der Laserstrahlung zur Datenempfangseinrichtung hin abgelenkt werden. Da es sich bei der Laserstrahlung um eine Laserstrahlung mit hoher Intensität handelt, ist dies auch notwendig, um die Datenempfangseinrichtung nicht zu beschädigen.

Die Beschichtung des dielektrischen Spiegels 330 ist derart, dass unter Berücksichtigung der Neigung des Spiegels zum Strahlengang der Laserstrahlung die Reflexion der Laserstrahlung minimiert ist, um zum einen die Verluste gering zu halten und zum anderen die umliegenden Elemente vor Schädigung durch Reflexe der intensiven Laserstrahlung zu bewahren. Vorzugsweise ist die Beschichtung des dielektrischen Spiegels auch dahingehend optimiert, dass für den Spektralbereich der von der Datensendeeinrichtung gesendeten optischen Impulse die Reflexion optimiert ist, um einen möglichst großen Anteil der optischen Signale an die Datenempfangseinrichtung weiterleiten zu können. Dadurch kann die Sendeleistung der Datensendeeinrichtung und damit der Strom- bzw. Energieverbrauch der Datensendeeinrichtung reduziert werden.

Die elektrischen Signale der Datenempfangseinrichtung 310 werden über eine elektronische Schaltung an die Anzeige- oder Regeleinrichtung oder an den Systemkontroller im Lasergerät 110 weitergeleitet, um die mittels der optischen Signale übertragenen Informationen in entsprechende Steuer- bzw. Regelprozesse umzuwandeln. Vorzugsweise werden mittels der optischen Signale der Datensendeeinrichtung Informationen zur Einstellung der Laserleistung übertragen.

Die Datenempfangseinrichtung 310 kann aus einem handelsüblichen lichtempfindlichen Sensor bestehen, der entweder für einen breiten Spektralbereich sensitiv ist oder nur für bestimmte Spektralbereiche.

Die bevorzugte Ausführungsform ist nicht auf eine Datenkommunikation nur vom Handstück zum stationären Lasergerät beschränkt. Sie kann auch für eine Datenkommunikation vom stationären Lasergerät zum Handstück verwendet werden.

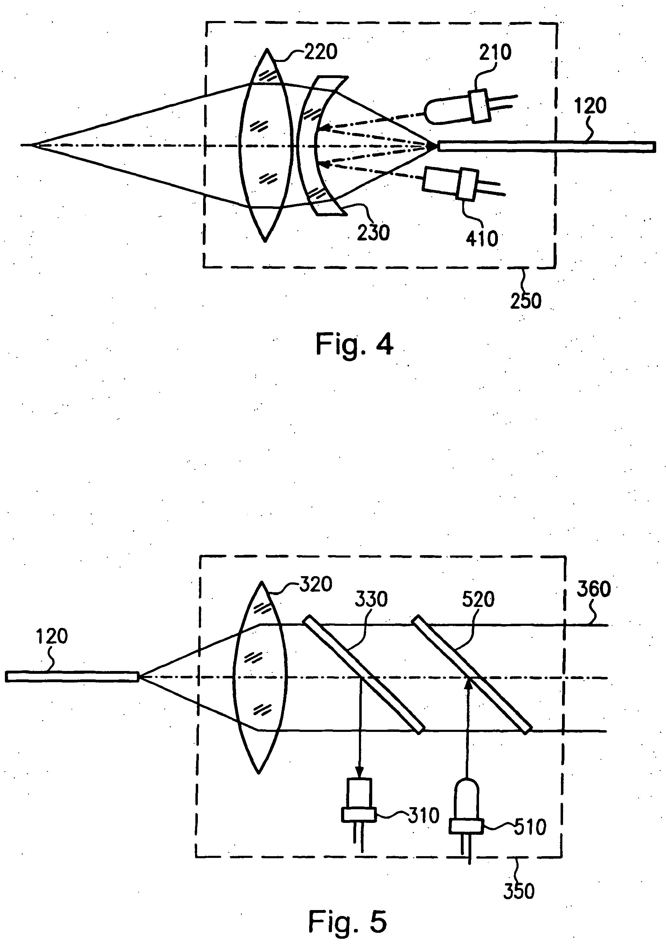

Fig. 4 zeigt anhand einer schematischen Darstellung die Auskoppeleinrichtung 250 einer besonders bevorzugten Ausführungsform der vorliegenden Erfindung. Bei dieser Ausführungsform wird zusätzlich zu der in Fig. 2 dargestellten Ausführungsform eine Datenempfangseinrichtung 410 an die Auskoppeleinrichtung gekoppelt. Die Datenempfangseinrichtung 410 ist derart positioniert, dass die aus dem Lichtleiter austretenden optischen Signale einer Datensendeeinrichtung am Linsensystem 220 und 230 reflektiert und in die Datenempfangseinrichtung abgelenkt werden. Auf diese Art wird verhindert, dass die Datenempfangseinrichtung den optischen Strahlengang der Laserstrahlung stört und zum anderen die Datenempfangseinrichtung selbst durch die direkte Laserstrahlung beschädigt bzw. gestört wird.

Die Datenempfangseinrichtung 410 kann aus einem handelsüblichen lichtempfindlichen Sensor bestehen, der alternativ für bestimmte Frequenzbereiche besonders sensitiv ist. Darüber hinaus kann die Datenempfangseinrichtung durch entsprechende optische Filter ergänzt werden, so dass nur optische Signale eines bestimmten Spektralbereichs in den Detektionsbereich der Datenempfangseinrichtung gelangen können. Die Datenempfangseinrichtung ist vorzugsweise über einen Handstückkontroller oder eine sonstige elektronische Schaltung mit einer entsprechenden Anzeigeeinrichtung im Handstück verbunden, mittels derer die über die optischen Signale übertragenen Informationen dargestellt werden können.

Umgekehrt wandelt die Datenempfangseinrichtung 310 die optischen Signale der Datensendeeinrichtung 210 in elektrische Signale um. Die elektrischen Signale werden über eine elektronische Schaltung an die Anzeige- oder Regeleinrichtung oder an den Systemkontroller im Lasergerät weitergeleitet, um die mittels der optischen Signale übertragenen Informationen in entsprechende Steuer- bzw. Regelprozesse umzuwandeln. Vorzugsweise werden mittels der optischen Signale der Datensendeeinrichtung Informationen zur Regelung der Laserleistung übertragen. Dies erleichtert die Handhabung des Lasersystems wesentlich, da alle wichtigen Informationen innerhalb des Blickfeldes des Anwenders liegen.

Die Anzeigeeinrichtung im Handstück kann z. B. dafür verwendet werden, die entsprechenden Anwendungsmodi anzuzeigen bzw. entsprechend des Verwendungszwecks des Lasers eine entsprechende Spotgröße der Laserstrahlung sowie die entsprechende Laserintensität anzuzeigen. Die notwendigen Informationen hierfür können von dem Lasergerät über eine entsprechende Datensendeeinrichtung 510 über den Lichtleiter an das Handstück übermittelt werden.

Fig. 5 zeigt die Einkoppeleinrichtung 350 der besonders bevorzugten Ausführungsform der vorliegenden Erfindung. Bei dieser besonders bevorzugten Ausführungsform wird zusätzlich zu der in Fig. 3 dargestellten Ausführungsform eine Datensendeeinrichtung 510 über einen dielektrischen Spiegel 520 in den Laserstrahl 360 eingekoppelt. Ähnlich wie der dielektrische Spiegel 330 ist der dielektrische Spiegel 520 gegen die Strahlrichtung der Laserstrahlung gekippt, um die von der Datensendeeinrichtung 510 gesendeten optischen Signale in den Strahlengang des Lasers einzukoppeln und über das Linsensystem 320 in den Lichtleiter 120 einzukoppeln. Die dielektrische Beschichtung des dielektrischen Spiegels 520 ist derart, dass die Reflexionsverluste der Laserstrahlung minimiert sind und dadurch die Transmission der Laserstrahlung durch den geneigten Spiegel optimiert ist. Vorzugsweise ist die dielektrische Beschichtung des Laserspiegels 520 auch dahingehend optimiert, dass der dielektrische Spiegel 520 für den Spektralbereich der von der Datensendeeinrichtung 510 erzeugten optischen Signale besonders gut reflektiert.

Alternativ kann für die Einkopplung der optischen Signale der Datensendeeinrichtung 510 auch der dielektrische Spiegel 330 verwendet werden, sowie das Linsensystem 320.

In der besonders bevorzugten Ausführungsform der vorliegenden Erfindung werden für die optischen Signale der Datensendeeinrichtung 210 und 510 verschiedene Spektralbereiche verwendet. Dadurch lassen sich zum einen die dielektrischen Beschichtungen der verwendeten optischen Komponenten bezüglich ihres Transmissions- und Reflexionsverhaltens optimieren, zum anderen kann die Sendeleistung für die Erzeugung der optischen Signale reduziert werden, was zur Energieeinsparung führt. Des Weiteren können für die optischen Signale der Datensendeeinrichtung 210 und 510 anstelle unterschiedlicher Spektralbereiche unterschiedliche Pulsfrequenzen verwendet werden, um einen ausreichenden Störabstand der erzeugten optischen Signale zu gewährleisten.

Zur Stromversorgung der Elektronik für die Datensendeeinrichtung 210 sowie für die Bedieneinrichtung 140 notwendige Elektronik verfügt das Handstück über eine Batterie bzw. über eine wiederaufladbare Batterie. Alternativ kann eine kapazitätsgepufferte Energieversorgung verwendet werden. Das Handstück muss zum einen ergonomische Anforderungen erfüllen, zum anderen muss es entsprechend ermüdungsfrei handhabbar sein, so dass das Gewicht des Handstücks möglichst gering gehalten werden muss. Daher wird in einer besonders bevorzugten Alternative der vorliegenden Ausführungsform eine energiesparende Elektronik verwendet, um das Gewicht für die Stromversorgung gering zu halten und trotzdem vernünftige Zeitdauern für die Verwendung und Bedienung des Handstücks zu gewährleisten.

In einer besonders bevorzugten Alternative der besonders bevorzugten Ausführungsform der vorliegenden Erfindung wird als Datensendeeinrichtung 510 ein Pilotlaser verwendet, der optische Signale im sichtbaren Spektralbereich erzeugt und zur Sichtbarmachung der im Lasergerät erzeugten Laserstrahlung dient. Zur Übertragung von Informationen über optische Signale wird der Pilotlaser vorzugsweise gepulst betrieben.

Alternativ werden zur Erzeugung der optischen Signale in der Datensendeeinrichtung 510 eine oder mehrere LEDs verwendet, die vorzugsweise gepulst betrieben werden, um einen genügenden Störabstand der erzeugten optischen Signale zu den anderen optischen Einflüssen zu gewährleisten.

Durch die Verwendung einer bestimmten Pulsfrequenz der optischen Signale können die übertragenen Informationen von Störeinflüssen bzw. von anderen optischen Signalen unterschieden werden. Dies gilt auch für die Datensendeeinrichtung 210, die in der besonders bevorzugten Alternative für die Übertragung der optischen Signale eine bestimmte Pulsfrequenz verwendet, damit sie von der Datenempfangseinrichtung 310 zuverlässig und mit entsprechendem Störabstand detektiert werden. Durch die Verwendung verschiedener Pulsdauer und verschiedener Frequenzen der optischen Signale wird dadurch eine zuverlässige bidirektionale Kommunikation zwischen Lasergerät und Handstück über den Lichtleiter realisiert, der vorrangig für die Übertragung intensiver Laserstrahlung verwendet wird.

Des Weiteren wird in der besonders bevorzugten Alternative der besonders bevorzugten Ausführungsform der vorliegenden Erfindung die Datenempfangseinrichtung 310 zur Messung von reemittierter Strahlung verwendet, die durch ein Bearbeiten von Material mittels der erzeugten Laserstrahlung entsteht. Hierbei wird der Effekt ausgenützt, dass die bei der Bearbeitung von Material erzeugte reemittierte Strahlung über das Linsensystem 220 und 230 in der Auskoppeleinrichtung in den Lichtwellenleiter 120 eingekoppelt wird und in der Einkoppeleinrichtung 250 zwangsläufig auch über den dielektrischen Spiegel 330 in die Datenempfangseinrichtung 310 reflektiert wird, da es sich hierbei im Allgemeinen um eine Strahlung mit einem breiten Spektralbereich handelt. Die Datenempfangseinrichtung 310 kann dabei die Intensität der reemittierten Strahlung messen und als elektrische Signale an den Systemkontroller weiterleiten. Entsprechend kann der Systemkontroller die Laserleistung regulieren und alternativ entsprechende Informationen über die Datensendeeinrichtung 510 an das Handstück senden.

Bei einer Anwendung im medizinischen Bereich ermöglicht die besonders bevorzugte Alternative der besonders bevorzugten Ausführungsform der vorliegenden Erfindung z. B. eine Variation der Laserspotgröße über die Bedienelemente 140 bei gleichzeitiger manueller oder automatischer Kontrolle der Lasertherapie über die reemittierte Strahlung, ohne dass zusätzliche elektrische Kabel vom Lasergerät zum Handstück geführt werden müssen oder störanfällige Funk- oder Infrarot-Sender verwendet werden müssen. Bevorzugterweise wird die Laserspotgröße, wie bereits oben erwähnt, automatisch erkannt, über eine Elektronik ausgewertet und die Laserleistung automatisch entsprechend nachgestellt.

In einer weiteren Alternative der besonders bevorzugten Ausführungsform der vorliegenden Erfindung ist das stationäre Lasergerät mit einer Ladestation für die wiederaufladbare Batterie im Handstück versehen. Die Ladestation ist derart gestaltet, dass es eine Halterung für eine lösbare Befestigung des Handstücks aufweist und sowohl in die Halterung als auch in das Handstück elektrische Kontakte integriert sind, mittels derer die wiederaufladbare Batterie geladen werden kann.

Um das Handstück entsprechend der unterschiedlichen Anwendungsmodi des Lasersystems flexibel einsetzen zu können, ist das Handstück modular aufgebaut und je nach Verwendungszweck des Lasersystems können Elemente des Handstücks ergänzt, ausgetauscht oder abgenommen werden. Alternativ verfügen die einzelnen Elemente des Handstücks über eine optisch, elektronisch oder mechanisch auswertbare ldentifizierungseinrichtung, die bei der Befestigung der Elemente am Handstück über eine Leseeinheit im Handstück ausgelesen wird und die Daten aus der Identifizierungseinrichtung an die Datensendeeinrichtung im Handstück weitergeleitet und zur Steuerung des Handstücks oder der Einrichtung zur Erzeugung von Laserstrahlung verwendet werden. Dies ermöglicht es, dass sich das Lasersystem automatisch auf bestimmte Anwendungsmodi einstellt und damit Behandlungsfehler - bedingt durch falsche Systemeinstellungen - minimiert werden.

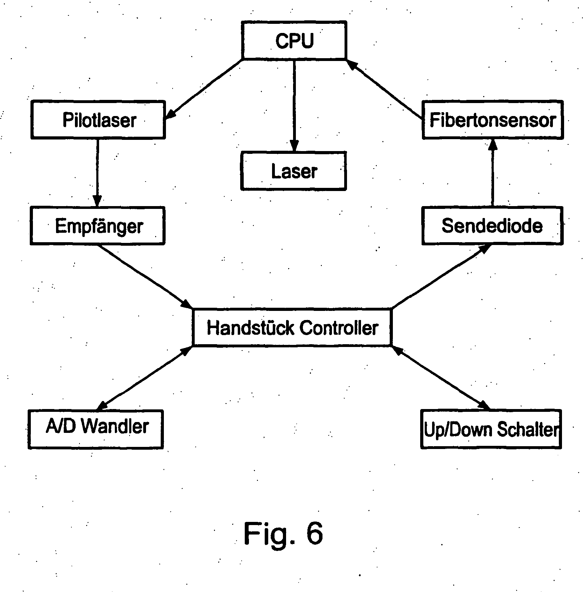

Fig. 6 zeigt ein Flussdiagramm zur Steuerung der Laserleistung gemäß einer Variante der besonders bevorzugten Ausführungsform der vorliegenden Erfindung. Hierbei ist die Elektronik im Handstück mit einem elektronischen Kontroller (Handstückkontroller) ausgestattet. Über die Bedieneinrichtungen 140 im Handstück mit den Tasten "up" und "down", werden Befehle zur Erhöhung oder zur Reduzierung der Laserleistung an den Handstückkontroller übermittelt. Der Handstückkontroller sendet die entsprechenden elektrischen Signale an die Datensendeeinrichtung 210 im Handstück. Dort werden sie in optische Signale umgewandelt, in den Lichtleiter 120 eingekoppelt und in der Einkoppeleinrichtung 350 von der Datenempfangseinrichtung 310 detektiert und in elektrische Signale umgewandelt. Elektrische Signale werden an den Systemkontroller weitergeleitet, der die Leistung des Lasers reduziert bzw. erhöht.

Den aktuellen Wert der Laserleistung übermittelt der Systemkontroller mittels elektrischer Pulse an den Pilotlaser, der entsprechende optische Signale über den Lichtleiter an die Datenempfangseinrichtung 410 im Handstück übermittelt, die dort detektiert werden und als elektrische Signale an den Handstückkontroller weitergeleitet werden. Dort wird dann z. B. der aktuelle Wert der Laserleistung in der Anzeigeeinrichtung im Handstück angezeigt. Alternativ meldet der Systemkontroller im Lasergerät auch die Intensität der gemessenen reemittierten Strahlung an den Handstückkontroller weiter.

Bevorzugterweise wird die Laserleistung direkt am Faserende ermittelt, da auf diese Weise die emittierte Laserleistung am zuverlässigsten bestimmt werden kann. Die Laserleistung wird zusätzlich in der Anzeigeeinrichtung im Handstück angezeigt. Die Anzeige der Werte erfolgt fallweise kontinuierlich oder ausschließlich bei Überschreitung eines bestimmten Wertes.

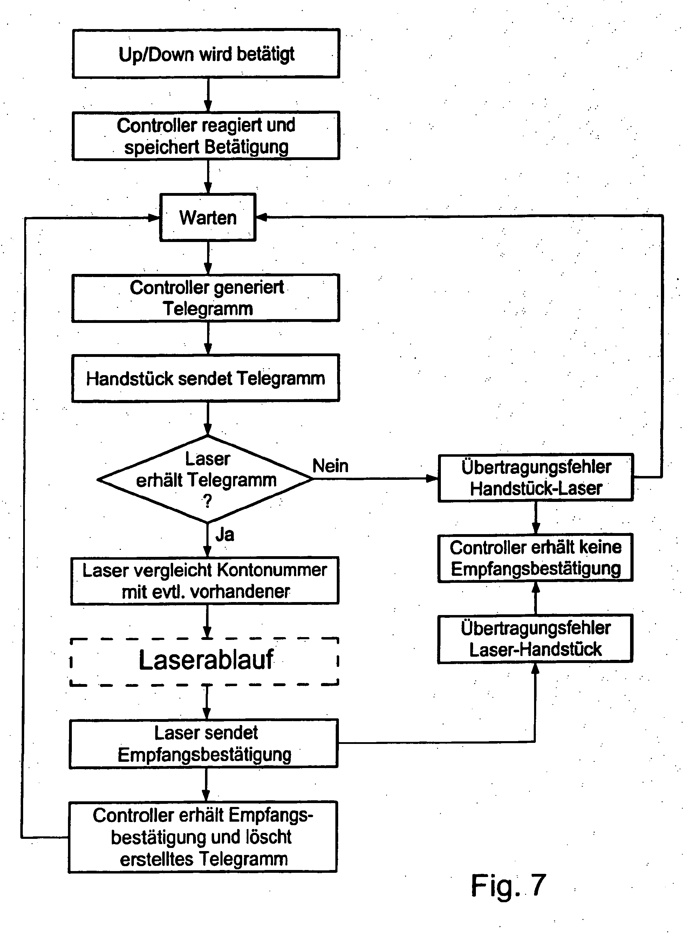

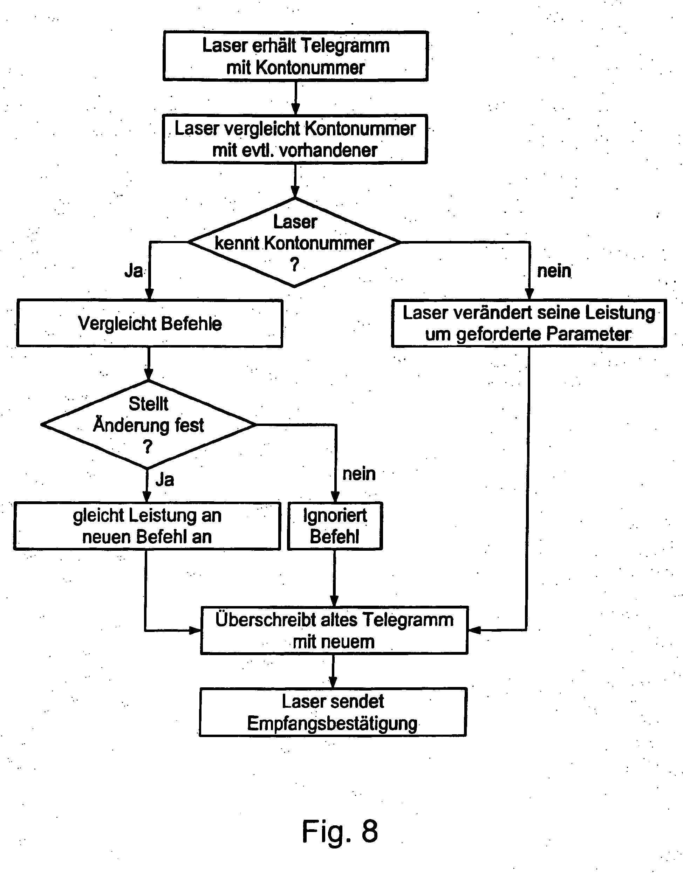

Figuren 7 und 8 zeigen anhand von Flussdiagrammen den in Fig. 6 beschriebenen Ablauf in Details. Durch die Betätigung der entsprechenden Bedieneinrichtung 140 werden entsprechende elektrische Signale an den Handstückkontroller übermittelt. Der Handstückkontroller wertet die elektrischen Signale aus und speichert sie in einem Zwischenspeicher. Daraufhin wartet der Handstückkontroller auf eine Systemanfrage vom Systemkontroller des Lasergeräts (Lasersystemkontroller) mittels des Pilotlasers 510. Erfolgt die Anfrage des Lasersystemkontrollers, generiert der Handstückkontroller ein sogenanntes Telegramm und sendet das Telegramm mittels der im Handstück befindlichen Datensendeeinrichtung 210 und dem Lichtleiter 120 an den Lasersystemkontroller. Erhält der Lasersystemkontroller das Telegramm, vergleicht der Lasersystemkontroller eine im Telegramm enthaltene ldentifizierungsnummer, auch Kontonummer genannt, mit bereits eventuell vorhandenen ldentifizierungsnummern. Ist die Sogenannte Kontonummer dem Lasersystemkontroller bekannt, werden die im Telegramm enthaltenen Steuerbefehle mit denen in dem bereits bekannten Telegramm verglichen. Werden Änderungen bei den Steuerbefehlen festgestellt, werden die aktuellen Steuerbefehle ausgeführt und das Telegramm mit der identischen Kontonummer überschrieben.

Ist dem Lasersystemkontroller die Kontonummer des übermittelten Telegramms noch nicht bekannt, wird die Überprüfung der Steuerbefehle übersprungen und die entsprechende Ausführung der Steuerbefehle sowie die Einstellung der notwendigen Parameter sofort vorgenommen.

Nach Ausführung der Steuerbefehle zur Einstellung der neuen Parameter sendet der Lasersystemkontroller eine Empfangsbestätigung mittels Pilotlaser 510 an den Handstückkontroller. Der Handstückkontroller empfängt die Empfangsbestätigung und löscht das Telegramm im Zwischenspeicher.

Über einen solchen Steuerkreis ist es nicht nur möglich Parameter des Therapielasers wie z. B. Laserleistung und Pulsdauer zu regeln, sondern auch optische Komponenten zu steuern, wie z. B. die Ein- und Auskoppeleinrichtung, um das Lasersystem an entsprechende Anwendungsmodi anzupassen.

Die vorliegende Erfindung ist nicht auf die aufgezählte bevorzugte Ausführungsform beschränkt, sondern erstreckt sich auch auf die Kombination aller bevorzugten Ausführungsfomnen.

Des Weiteren beschränkt sich die vorliegende Erfindung nicht auf den Bereich der medizinischen Anwendung, sondern kann äquivalent für Bereiche der Materialbearbeitung sowie Materialanalyse verwendet werden.

## Patentansprüche

1. Lasersystem (100), mit:
einer Einrichtung (110) zur Erzeugung von Laserstrahlung, die für medizinische Anwendungen und/oder für Bereiche der Materialbearbeitung und/oder für Bereiche der Materialanalyse verwendet werden kann;
einer Einkoppeleinrichtung (350) zur Einkopplung der erzeugten Laserstrahlung in einen Lichtleiter (120); und
einer Auskoppeleinrichtung (250) zur Auskopplung der Laserstrahlung aus dem Lichtleiter,
**dadurch gekennzeichnet, dass** eine Datensendeeinrichtung (210, 510) an die Ein-oder Auskoppeleinrichtung gekoppelt ist und optische Signale erzeugt, die über die Ein- oder Auskoppeleinrichtung in den Lichtleiter eingekoppelt werden und am anderen Ende des Lichtleiters von einer Datenempfangseinrichtung (310, 410) empfangen werden, wobei die Datensendeeinrichtung, falls diese an die Auskoppeleinrichtung gekoppelt ist, eine Lichtquelle ist.

2. Lasersystem nach Anspruch 1, **dadurch gekennzeichnet, dass** die Datensendeeinrichtung (210) an die Auskoppeleinrichtung (250) und die Datenempfangseinrichtung (310) an die Einkoppeleinrichtung (350) gekoppelt ist und die optischen Signale von der Einkoppeleinrichtung aus dem Strahlengang der Laserstrahlung ausgekoppelt werden.

3. Lasersystem nach Anspruch 1, **dadurch gekennzeichnet, dass** die Datensendeeinrichtung (510) an die Einkoppeleinrichtung (350) und die Datenempfangseinrichtung (410) an die Auskoppeleinrichtung (250) gekoppelt ist und die optischen Signale von der Auskoppeleinrichtung aus dem Strahlengang der Laserstrahlung ausgekoppelt werden.

4. Lasersystem nach Anspruch 2, **dadurch gekennzeichnet, dass** eine zusätzliche Datensendeeinrichtung (510) an die Einkoppeleinrichtung (350) sowie eine zusätzliche Datenempfangseinrichtung (410) an die Auskoppeleinrichtung (250) gekoppelt ist und die zusätzliche Datensendeeinrichtung optische Signale an die zusätzliche Datenempfangseinrichtung sendet und somit eine bidirektionale, optische Signalübertragung über den Lichtleiter ermöglicht wird.

5. Lasersystem nach Anspruch 4, **dadurch gekennzeichnet, dass** die Wellenlängen der optischen Signale der beiden Datensendeeinrichtungen unterschiedlich sind.

6. Lasersystem nach einem der Ansprüche 1 bis 5, **dadurch gekennzeichnet, dass** die Wellenlänge der erzeugten Laserstrahlung und die Wellenlänge der optischen Signale der Datensendeeinrichtung unterschiedlich sind.

7. Lasersystem nach einem der Ansprüche 1 bis 6, **dadurch gekennzeichnet, dass** die optischen Signale zur Übertragung von Informationen und insbesondere zur Steuerung der Vorrichtung zur Erzeugung von Laserstrahlung oder zur Steuerung der Einkoppeleinrichtung dienen.

8. Lasersystem nach einem der Ansprüche 1 bis 7, **dadurch gekennzeichnet, dass** die optischen Signale insbesondere zur Steuerung einer Leistung der erzeugten Laserstrahlung dienen.

9. Lasersystem nach einem der Ansprüche 1 bis 8, **dadurch gekennzeichnet, dass** die erzeugte Laserstrahlung eine Laserstrahlung mit hoher Leistung ist und damit für Anwendungen in der Medizintechnik geeignet ist.

10. Lasersystem nach einem der Ansprüche 1 bis 9, **dadurch gekennzeichnet, dass** die Auskoppeleinrichtung ein Linsensystem (220, 230) einer Fokussiereinheit für die Laserstrahlung beinhaltet und die optischen Signale über das Linsensystem eingekoppelt werden.

11. Lasersystem nach einem der Ansprüche 1 bis 10, **dadurch gekennzeichnet, dass** die an die Auskoppeleinrichtung gekoppelte Datensendeeinrichtung eine LED (210) zur Erzeugung der optischen Signale aufweist.

12. Lasersystem nach Anspruch 11, **dadurch gekennzeichnet, dass** die zur Erzeugung der optischen Signale verwendete LED optischen Signale im blauen Spektralbereich erzeugt.

13. Lasersystem nach einem der Ansprüche 1 bis 12, **dadurch gekennzeichnet, dass** das Linsensystem der Fokussiereinheit eine dielektrische Beschichtung aufweist, die ein besonders effizientes Einkoppeln der optischen Signale über das Linsensystem ermöglicht.

14. Lasersystem nach einem der Ansprüche 1 bis 13, **dadurch gekennzeichnet, dass** das Auskoppeln der optischen Signale über einen Spiegel (330) mit dielektrischer Beschichtung in der Einkoppeleinrichtung erfolgt, der für die erzeugte Laserstrahlung transparent ist und die optischen Signale reflektiert.

15. Lasersystem nach einem der Ansprüche 1 bis 14, **dadurch gekennzeichnet, dass** die optischen Signale der Datensendeeinrichtungen bestimmte Pulsdauern aufweisen, um die optischen Signale voneinander und von Störsignalen unterscheiden zu können.

16. Lasersystem nach einem der Ansprüche 1 bis 15, **dadurch gekennzeichnet, dass** die an die Einkoppeleinrichtung (350) gekoppelte Datenempfangseinrichtung (310) auch zur Detektion weiterer optischer Signale verwendet wird.

17. Lasersystem nach einem der Ansprüche 1 bis 16, **dadurch gekennzeichnet, dass** das Lasersystem ein medizinisches Lasersystem ist.

18. Lasersystem nach einem der Ansprüche 1 bis 17, **dadurch gekennzeichnet, dass** das Lasersystem über eine Einrichtung zur Messung von reemittierter Strahlung verfügt, die durch ein Bearbeiten von Material mittels der erzeugten Laserstrahlung entsteht.

19. Lasersystem nach Anspruch 18, **dadurch gekennzeichnet, dass** die reemittierte Strahlung über die an die Einkoppeleinrichtung gekoppelte Datenempfangseinrichtung (310) detektiert wird.

20. Lasersystem nach einem der Ansprüche 4 bis 19 mit Rückbezug auf Anspruch 4, **dadurch gekennzeichnet, dass** das Lasersystem (100) zusätzlich ein Handstück (130) beinhaltet, das Handstück über den Lichtleiter (120) mit einer Einrichtung (110) zur Erzeugung von Laserstrahlung verbunden ist und die Auskoppeleinrichtung (250) zur Auskopplung Laserstrahlung aus dem Lichtleiter sowie die Datensendeeinrichtung (210) und die Datenempfangseinrichtung (410) in dem Handstück integriert sind.

21. Lasersystem nach Anspruch 20, **dadurch gekennzeichnet, dass** das Handstück Bedieneinrichtungen (140) aufweist, die bei Betätigung elektrische Signale erzeugen und an die Datensendeinrichtungen im Handstück weiterleiten, mittels derer die Einrichtung (110) zur Erzeugung von Laserstrahlung gesteuert wird.

22. Lasersystem nach einem der Ansprüche 20 bis 21, **dadurch gekennzeichnet, dass** das Handstück eine Anzeigeeinrichtung (160) aufweist, die Informationen anzeigt, die über die an die Auskoppeleinrichtung gekoppelte Datenempfangseinrichtung empfangen werden.

23. Lasersystem nach einem der Ansprüche 20 bis 22, **dadurch gekennzeichnet, dass** im Handstück eine wiederaufladbare Batterie zur Stromversorgung der Datensende-und Datenempfangseinrichtung sowie der Bedien- und Anzeigeeinrichtungen integriert ist.

24. Lasersystem nach Anspruch 23, **dadurch gekennzeichnet, dass** die wiederaufladbare Batterie im Handstück über eine an die Einrichtung zur Erzeugung von Laserstrahlung angeschlossene Ladestation wiederaufgeladen wird.

25. Lasersystem nach Anspruch 24, **dadurch gekennzeichnet, dass** die Ladestation eine Halterung für eine lösbare Befestigung des Handstücks aufweist und sowohl in die Halterung als auch in das Handstück elektrische Kontakte integriert sind, mittels derer die wiederaufladbare Batterie geladen werden kann.

26. Lasersystem nach einem der Ansprüche 23 bis 25, **dadurch gekennzeichnet, dass** im Handstück alternativ oder zusätzlich zu den wiederaufladbaren Batterien Photozellen zur Stromversorgung der Datensende- und Datenempfangseinrichtung sowie der Bedien- und Anzeigeeinrichtungen integriert sind, die Raumlicht und/oder reflektierte Laserstrahlung zur Erzeugung elektrischer Energie verwenden.

27. Lasersystem nach einem der Ansprüche 20 bis 26, **dadurch gekennzeichnet, dass** das Handstück modular aufgebaut ist und je nach Verwendungszweck des Lasersystems Elemente des Handstücks ergänzt, ausgetauscht oder abgenommen werden.

28. Lasersystem nach Anspruch 27, **dadurch gekennzeichnet, dass** die einzelnen Elemente des Handstücks über eine elektronisch, optisch oder mechanisch auswertbare Identifizierungseinrichtung verfügen, die bei der Befestigung der Elemente am Handstück über eine Leseeinheit im Handstück ausgelesen wird und Daten aus der Identifizierungseinrichtung an die Datensendeinrichtung im Handstück weitergeleitet und zur Steuerung des Handstücks oder der Einrichtung zur Erzeugung von Laserstrahlung verwendet werden.

## Claims

1. Laser system (100), with:
a device (110) for producing laser radiation which can be used for medical applications and/or for areas in material processing and/or for areas in material analysis;
an incoupling device (350) for incoupling the laser radiation produced into a light guide (120); and
an outcoupling device (250) for outcoupling the laser radiation from the light guide,
**characterised in that** a data transmitting device (210, 510) is coupled to the incoupling device or outcoupling device and produces optical signals which are incoupled into the light guide via the incoupling device or outcoupling device and are received at the other end of the light guide by a data receiving device (310, 410), wherein the data transmitting device, if it is coupled to the outcoupling device, is a light source.

2. Laser system according to claim 1, **characterised in that** the data transmitting device (210) is coupled to the outcoupling device (250) and the data receiving device (310) is coupled to the incoupling device (350) and the optical signals are outcoupled from the optical path of the laser radiation by the incoupling device.

3. Laser system according to claim 1, **characterised in that** the data transmitting device (510) is coupled to the incoupling device (350) and the data receiving device (410) is coupled to the outcoupling device (250) and the optical signals are outcoupled from the optical path of the laser radiation by the outcoupling device.

4. Laser system according to claim 2, **characterised in that** an additional data transmitting device (510) is coupled to the incoupling device (350) and an additional data receiving device (410) is coupled to the outcoupling device (250) and the additional data transmitting device sends optical signals to the additional data receiving device and thus a bidirectional, optical signal transmission via the light guide is made possible.

5. Laser system according to claim 4, **characterised in that** the wavelengths of the optical signals of the two data transmitting devices are different.

6. Laser system according to one of claims 1 to 5, **characterised in that** the wavelength of the laser radiation produced and the wavelength of the optical signals of the data transmitting device are different.

7. Laser system according to one of claims 1 to 6, **characterised in that** the optical signals are used for transmitting information and in particular for controlling the device for producing laser radiation or for controlling the incoupling device.

8. Laser system according to one of claims 1 to 7, **characterised in that** the optical signals are used in particular for controlling a power of the laser radiation produced.

9. Laser system according to one of claims 1 to 8, **characterised in that** the laser radiation produced is a high-power laser radiation and therefore is suitable for applications in medical technology.

10. Laser system according to one of claims 1 to 9, **characterised in that** the outcoupling device contains a lens system (220, 230) of a focusing unit for the laser radiation and the optical signals are incoupled via the lens system.

11. Laser system according to one of claims 1 to 10, **characterised in that** the data transmitting device coupled to the outcoupling device has a LED (210) for producing the optical signals.

12. Laser system according to claim 11, **characterised in that** the LED used to produce the optical signals produces optical signals in the blue spectral range.

13. Laser system according to one of claims 1 to 12, **characterised in that** the lens system of the focussing unit has a dielectric coating which makes possible a particularly efficient incoupling of the optical signals via the lens system.

14. Laser system according to one of claims 1 to 13, **characterised in that** the outcoupling of the optical signals is carried out in the incoupling device via a mirror (330) with dielectric coating, which mirror is transparent to the laser radiation produced and reflects the optical signals.

15. Laser system according to one of claims 1 to 14, **characterised in that** the optical signals of the data transmitting devices have specific pulse durations in order to be able to distinguish the optical signals from one another and from interfering signals.

16. Laser system according to one of claims 1 to 15, **characterised in that** the data receiving device (310) coupled to the incoupling device (350) is also used for the detection of further optical signals.

17. Laser system according to one of claims 1 to 16, **characterised in that** the laser system is a medical laser system.

18. Laser system according to one of claims 1 to 17, **characterised in that** the laser system has a device for measuring reemitted radiation which is produced by a treatment of material by means of the laser radiation produced.

19. Laser system according to claim 18, **characterised in that** the reemitted radiation is detected via the data receiving device (310) coupled to the incoupling device.

20. Laser system according to one of claims 4 to 19 relating to claim 4, **characterised in that** the laser system (100) additionally contains a handpiece (130), the handpiece is connected via the light guide (120) to a device (110) for producing laser radiation and the outcoupling device (250) for outcoupling laser radiation from the light guide and the data transmitting device (210) and the data receiving device (410) are integrated in the handpiece.

21. Laser system according to claim 20, **characterised in that** the handpiece has operating devices (140) which on actuation produce electrical signals and forward to the data transmitting device in the handpiece, by means of which the device (110) for producing laser radiation is controlled.

22. Laser system according to one of claims 20 to 21, **characterised in that** the handpiece has a display device (160) which displays information which is received via the data receiving device coupled to the outcoupling device.

23. Laser system according to one of claims 20 to 22, **characterised in that** a rechargeable battery for supplying power to the data transmitting device and data receiving device and to the operating and display devices is integrated in the handpiece.

24. Laser system according to claim 23, **characterised in that** the rechargeable battery in the handpiece is recharged via a charging station connected to the device for producing laser radiation.

25. Laser system according to claim 24, **characterised in that** the charging station has a holder for detachable attaching of the handpiece and electrical contacts, by means of which the rechargeable battery can be charged, are integrated both in the holder and in the handpiece.

26. Laser system according to one of claims 23 to 25, **characterised in that** photocells for supplying power to the data transmitting device and data receiving device and to the operating and display devices are integrated in the handpiece alternatively or additionally to the rechargeable batteries, which photocells use ambient light and/or reflected laser radiation to produce electrical energy.

27. Laser system according to one of claims 20 to 26, **characterised in that** the handpiece has a modular structure and depending on the purpose of the laser system, elements of the handpiece are supplemented, replaced or removed.

28. Laser system according to claim 27, **characterised in that** the individual elements of the handpiece have an identification device which can be evaluated electronically, optically or mechanically, which is read via a reading unit in the handpiece when the elements are attached to the handpiece, and data from the identification device is forwarded to the data transmitting device in the handpiece and is used to control the handpiece or the device for producing laser radiation.

## Revendications

1. Système laser (100), avec :
un dispositif (110) pour générer un rayonnement laser, lequel peut être utilisé pour des applications médicales et/ou des domaines de traitement des matériaux et/ou des domaines d'analyse de matériaux ;
un dispositif d'injection (350) pour l'injection du rayonnement laser généré dans un câble à fibre optique (120) ; et
un dispositif de déclenchement (250) pour le déclenchement du rayonnement laser issu du câble à fibre optique,
**caractérisé en ce qu'**un dispositif émetteur de données (210, 510) est couplé au dispositif d'injection ou de déclenchement et génère des signaux optiques, lesquels sont injectés via le dispositif d'injection ou de déclenchement dans le câble à fibre optique et sont réceptionnés à l'autre extrémité du câble à fibre optique par un dispositif de réception de données (310, 410), le dispositif émetteur de données, s'il est couplé au dispositif de déclenchement, étant une source lumineuse.

2. Système laser selon la revendication 1, **caractérisé en ce que** le dispositif émetteur de données (210) est couplé au dispositif de déclenchement (250) et le dispositif de réception de données (310) au dispositif d'injection (350), et **en ce que** les signaux optiques sont déclenchés par le dispositif d'injection à partir du trajet du rayonnement du rayonnement laser.

3. Système laser selon la revendication 1, **caractérisé en ce que** le dispositif émetteur de données (510) est couplé au dispositif d'injection (350) et le dispositif de réception de données (410) au dispositif de déclenchement (250), et **en ce que** les signaux optiques sont déclenchés par le dispositif de déclenchement à partir du trajet du rayonnement du rayonnement laser.

4. Système laser selon la revendication 2, **caractérisé en ce qu'**un dispositif émetteur de données supplémentaire (510) est couplé au dispositif d'injection (350) ainsi qu'un dispositif de réception de données supplémentaire (410) au dispositif de déclenchement (250), et **en ce que** le dispositif émetteur de données supplémentaire émet des signaux optiques à l'attention du dispositif de réception de données supplémentaire de sorte que de cette manière, une transmission de signaux optiques bidirectionnelle devient possible via le câble à fibre optique.

5. Système laser selon la revendication 4, **caractérisé en ce que** les longueurs d'onde des signaux optiques des deux dispositifs émetteurs de données sont différentes.

6. Système laser selon l'une quelconque des revendications 1 à 5, **caractérisé en ce que** les longueurs d'onde du rayonnement laser généré et les longueurs d'ondes des signaux optiques du dispositif émetteur de données sont différentes.

7. Système laser selon l'une quelconque des revendications 1 à 6, **caractérisé en ce que** les signaux optiques servent à la transmission d'informations et en particulier à la commande du dispositif pour la génération d'un rayonnement laser ou à la commande du dispositif d'injection.

8. Système laser selon l'une quelconque des revendications 1 à 7, **caractérisé en ce que** les signaux optiques servent en particulier à la commande d'une puissance du rayonnement laser généré.

9. Système laser selon l'une quelconque des revendications 1 à 8, **caractérisé en ce que** le rayonnement laser généré est un rayonnement laser à puissance élevée et convient par conséquent pour des applications de techniques médicales.

10. Système laser selon l'une quelconque des revendications 1 à 9, **caractérisé en ce que** le dispositif de déclenchement comprend un système de lentilles (220, 230) d'une unité de focalisation pour le rayonnement laser, et **en ce que** les signaux optiques sont injectés via le système de lentilles.

11. Système laser selon l'une quelconque des revendications 1 à 10, **caractérisé en ce que** le dispositif émetteur de données couplé au dispositif de déclenchement présente une DEL (210) pour générer les signaux optiques.

12. Système laser selon la revendication 11, **caractérisé en ce que** la DEL utilisée pour générer les signaux optiques génère des signaux optiques dans le domaine spectral du bleu.

13. Système laser selon l'une quelconque des revendications 1 à 12, **caractérisé en ce que** le système de lentilles de l'unité de focalisation présente un revêtement diélectrique lequel permet une injection particulièrement efficace des signaux optiques via le système de lentilles.

14. Système laser selon l'une quelconque des revendications 1 à 13, **caractérisé en ce que** le déclenchement des signaux optiques est réalisé par l'intermédiaire d'un miroir (330) à revêtement diélectrique dans le dispositif d'injection, lequel est transparent pour le rayonnement laser généré et réfléchit les signaux optiques.

15. Système laser selon l'une quelconque des revendications 1 à 14, **caractérisé en ce que** les signaux optiques du dispositifs émetteur de données présentent des durées d'impulsion déterminées afin de pouvoir distinguer les signaux optiques les uns des autres et des signaux parasites.

16. Système laser selon l'une quelconque des revendications 1 à 15, **caractérisé en ce que** le dispositif de réception de données (310) couplé au dispositif d'injection (350) est également utilisé pour la détection d'autres signaux optiques.

17. Système laser selon l'une quelconque des revendications 1 à 16, **caractérisé en ce que** le système laser est un système laser médical.

18. Système laser selon l'une quelconque des revendications 1 à 17, **caractérisé en ce que** le système laser dispose d'un dispositif pour mesurer un rayonnement réémis, lequel est obtenu grâce à un traitement de matériau à l'aide du rayonnement laser généré.

19. Système laser selon la revendication 18, **caractérisé en ce que** le rayonnement réémis est détecté grâce au dispositif de réception de données (310) couplé au dispositif d'injection.

20. Système laser selon l'une quelconque des revendications 4 à 19 en se référant à la revendication 4, **caractérisé en ce que** le système laser (100) comprend de manière supplémentaire une partie tenue en main (130), la partie tenue en main étant reliée via le câble à fibre optique (120) à un dispositif (110) pour la génération d'un rayonnement laser, et **en ce que** le dispositif de déclenchement (250) pour le déclenchement du rayonnement laser issu du câble à fibre optique ainsi que le dispositif émetteur de données (210) et le dispositif de réception de données (410) sont intégrés dans la partie tenue en main.

21. Système laser selon la revendication 20, **caractérisé en ce que** la partie tenue en main présente des dispositifs de commande (140) lesquels génèrent des signaux électriques en cas d'actionnement et les retransmettent aux dispositifs émetteurs de données dans la partie tenue en main, grâce auxquels on commande le dispositif (110) pour la génération d'un rayonnement laser.

22. Système laser selon l'une quelconque des revendications 20 à 21, **caractérisé en ce que** la partie tenue en main présente un dispositif d'affichage (160), lequel affiche des informations, lesquelles sont réceptionnées par l'intermédiaire du dispositif de réception de données couplé au dispositif de déclenchement.

23. Système laser selon l'une quelconque des revendications 20 à 22, **caractérisé en ce qu'**une pile rechargeable pour l'alimentation électrique du dispositif émetteur de données et de réception de données ainsi que des dispositifs de commande et d'affichage est intégrée dans la partie tenue en main.

24. Système laser selon la revendication 23, **caractérisé en ce que** la pile rechargeable dans la partie tenue en main est rechargée grâce à une station de charge raccordée au dispositif pour la génération d'un rayonnement laser.

25. Système laser selon la revendication 24, **caractérisé en ce que** la station de charge présente une fixation pour une fixation détachable de la partie tenue en main, et **en ce que** des contacts électriques sont intégrés dans la fixation tout comme également dans la partie tenue en main, à l'aide desquels la pile rechargeable peut être chargée.

26. Système laser selon l'une quelconque des revendications 23 à 25, **caractérisé en ce que** l'on intègre dans la partie tenue en main, en variante ou de manière supplémentaire aux piles rechargeables, des photocellules pour l'alimentation en courant du dispositif émetteur de données et de réception de données ainsi que des dispositifs de commande et d'affichage, lesquelles utilisent la lumière de la pièce et/ou un rayonnement laser réfléchi pour la génération d'énergie électrique.

27. Système laser selon l'une quelconque des revendications 20 à 26, **caractérisé en ce que** la partie tenue en main est de configuration modulaire, et **en ce qu'**en fonction de l'usage du système laser, des éléments de la partie tenue en main sont rajoutés, échangés ou retirés.

28. Système laser selon la revendication 27, **caractérisé en ce que** les éléments individuels de la partie tenue en main disposent d'un dispositif d'identification pouvant être analysé électroniquement, optiquement ou mécaniquement, lequel est lu via une unité de lecture lors de la fixation des éléments sur la partie tenue en main, et **en ce que** des données issues du dispositif d'identification sont retransmises au dispositif émetteur de données dans la partie tenue en main et sont utilisées pour la commande de la partie tenue en main ou du dispositif pour la génération d'un rayonnement laser.
